# EUROPÄISCHE PATENTSCHRIFT

(11) **EP 3 618 710 B1**
(45) Veröffentlichungstag und Bekanntmachung des Hinweises auf die Patenterteilung: **17.08.2022**
(21) Anmeldenummer: 18722002.5
(22) Anmeldetag: 26.04.2018
(51) Int. Cl.: A61B 5/08, G01N 33/497

(54) **VORRICHTUNG, SYSTEM UND VERFAHREN ZUR BEREITSTELLUNG EINES KALIBRIERFLUIDS FÜR EINE KALIBRIERUNG EINER ATEMGASANALYSEEINRICHTUNG**
DEVICE, SYSTEM, AND METHOD FOR PROVIDING A CALIBRATION FLUID FOR THE CALIBRATION OF A RESPIRATORY GAS ANALYSIS DEVICE
DISPOSITIF, SYSTÈME ET PROCÉDÉ POUR PRÉPARER UN FLUIDE D'ÉTALONNAGE POUR UN ÉTALONNAGE DE QUALITÉ D'UN DISPOSITIF D'ANALYSE DE GAZ RESPIRATOIRE

(30) Priorität: 03.05.2017 DE 102017207429
(43) Veröffentlichungstag der Anmeldung: 11.03.2020
(73) Patentinhaber: Robert Bosch GmbH, 70442 Stuttgart (DE)
(72) Erfinder: SAHNER, Kathy, 68167 Mannheim (DE); GRANER, Kathrin, 71334 Waiblingen (DE); JANK, Heike, 71394 Kernen Im Remstal (DE); LAUTERBACH, Sonja, 71332 Waiblingen (DE)
(86) Internationale Anmeldenummer: PCT/EP2018/060739
(87) Internationale Veröffentlichungsnummer: WO 2018/202543

(56) Entgegenhaltungen:
- DE-A1- 10 137 565
- DE-A1-102009 056 338
- DE-A1-102014 108 109
- DE-C1- 3 642 609

## Beschreibung

### Stand der Technik

Über quantitative Messungen von Analyten in Ausatemluft können bestimmte Atemwegserkrankungen erkannt und überwacht werden. Beispielsweise kann über die Bestimmung der Stickstoffmonoxidkonzentration in ausgeatmeter Luft ein Maß für die Entzündung der Lunge bei Asthma abgeschätzt werden. Die Bestimmung der Stickstoffmonoxidkonzentration kann dabei mit einer in EP 1384069 B1 offenbarten Vorrichtung über die Konversion von Stickstoffmonoxid zu Stickstoffdioxid mit nachfolgender Messung der Stickstoffdioxidkonzentration mithilfe eines feldeffekttransistorbasierten Gassensors in der Vorrichtung erfolgen. Der Gassensor weist dazu auf seiner Gate-Elektrode eine sensitive Schicht zur Anlagerung von Stickstoffdioxid auf. Für die Regeneration des Gassensors und insbesondere der sensitiven Schicht nach einer Messung ist eine Spülung bei Erhitzung der sensitiven Schicht erforderlich. Eine vollständige Entfernung von auf der sensitiven Schicht angelagertem Stickstoffdioxid und anderen, die Messung störenden Spezies ist dabei jedoch in der Regel nicht möglich.

Dem Stand der Technik entsprechende Vorrichtungen zur Bereitstellung von Kalibrierfluiden für Gasanalysevorrichtungen werden zum Beispiel in DE3642609C1, DE10137565A1 und DE102014108109A1 beschrieben.

### Offenbarung der Erfindung

### Vorteile der Erfindung

Vor diesem Hintergrund betrifft die Erfindung eine Vorrichtung, ein System und ein Verfahren zur Bereitstellung eines Kalibrierfluids, insbesondere eines Stickstoffoxids, für eine Kalibrierung einer Atemgasanalyseeinrichtung.

Die erfindungsgemäße Vorrichtung zur Bereitstellung eines Kalibrierfluids ist im Anspruch 1 definiert.

Unter einem Kalibrierfluid ist insbesondere ein Gas oder ein Gasgemisch mit vorgegebener Zusammensetzung zu verstehen, so dass unter Einsatz des Kalibrierfluids eine wohldefinierte Kalibrierung der Atemgasanalyseeinrichtung möglich ist. Das Kalibrierfluid kann Stickstoffoxide, insbesondere Stickstoffmonoxid und/oder Stickstoffdioxid umfassen. Unter einer Atemgasanalyseeinrichtung ist insbesondere eine Vorrichtung zur Messung von Analyten in Ausatemluft zu verstehen, auch Gerät zur Atemgasanalyse genannt, beispielsweise eine in EP 1384069 B1 offenbarte Vorrichtung zur Messung von Stickstoffmonoxid in Ausatemluft basierend auf einer Konversion von Stickstoffmonoxid zu Stickstoffdioxid und einer Messung der Stickstoffdioxidkonzentration. Bei der ersten Schnittstelle kann es sich insbesondere um einen Anschluss für die Atemgasanalyseeinrichtung handeln, beispielsweise um einen Teil einer insbesondere formschlüssigen Verbindung zu der Atemgasanalyseeinrichtung, beispielsweise einen Teil einer Clipverbindung oder einen Teil einer Steckverbindung. Die erste Schnittstelle kann dabei eine verschließbare Öffnung zwischen der Kammer der Vorrichtung und einem Bereich außerhalb der Vorrichtung aufweisen. Bei der Kammer handelt es sich insbesondere um eine quaderförmige, beispielsweise würfelförmige, oder zylinderförmige Ausnehmung in der Vorrichtung, wobei das Volumen der Ausnehmung für ein wohldefiniertes Volumen des in der Kammer zu erzeugenden Kalibrierfluids, insbesondere Kalibriergases, vorgegeben ist. Die erste Öffnung kann insbesondere als Schlitz für das Einbringen eines streifenförmigen Reaktantenträgers ausgebildet sein. Unter dem Kontaktelement ist insbesondere eine in der Kammer angeordnete Struktur zu verstehen. Insbesondere ist das Kontaktelement für eine Auslösung der chemischen Reaktion mit dem ersten Reaktanten zur Erzeugung des Kalibrierfluids bei über die erste Öffnung eingebrachtem Reaktantenträger ausgebildet.

Die erfindungsgemäße Vorrichtung hat den Vorteil, dass die Atemgasanalyseeinrichtung auf einfache Art und Weise nach vorangegangenem Gebrauch neu kalibriert werden kann. Darüber hinaus kann vorteilhafterweise die Wirkung einer vorangegangenen Spülung des Gassensor der Atemgasanalyseeinrichtung überprüft werden. Über die Vorgabe der chemischen Reaktion zur Erzeugung des Kalibrierfluids und der daran beteiligten Mengen an Reaktanten sowie über das wohldefinierte Volumen der Kammer der Vorrichtung kann der Atemgasanalyseeinrichtung eine wohldefinierte Konzentration an Kalibrierfluid für die Kalibriermessung zugeführt werden. Somit ermöglicht die Erfindung vorteilhafterweise einen einfach durchzuführenden Vergleich der gemessenen Konzentration mit der vorgegebenen Konzentration für eine Überprüfung der Messgenauigkeit. Dadurch kann auch eine Qualitätskontrolle der Vorrichtung auf einfache Weise durchgeführt werden, insbesondere bei einem Einsatz der Vorrichtung im Feld. Ferner ist von Vorteil, dass für eine Bereitstellung des Kalibrierfluids kein Anschluss an eine Fluidleitung oder an einen Vorratsbehälter für Fluid vorgesehen werden muss, insbesondere kein Anschluss an eine Gasleitung beziehungsweise eine Druckgasflache. Da insbesondere kein Vorratsbehälter für Fluid benötigt wird und die Erzeugung des Kalibrierfluids erst bei tatsächlichem Bedarf erfolgen kann, kann die Vorrichtung vorteilhafterweise kompakt und platzsparend ausgeführt sein. Die Erzeugung des Kalibrierfluids erst bei tatsächlichem Bedarf hat darüber hinaus den Vorteil, dass das Kalibrierfluid immer frisch erzeugt werden kann und somit gegenüber einem gelagerten Kalibrierfluid in der Regel einer höhere Qualität und/oder Stabilität aufweist.

Vorzugsweise umfasst das Kontaktelement eine Struktur, insbesondere einen Vorsprung, zur Kontaktierung des eingebrachten Reaktantenträgers. Die Struktur kann dabei derart bezüglich der ersten Öffnung angeordnet und ausgebildet sein, dass sie eine Einbringung des Reaktantenträgers in die Kammer auf ein vorgegebenes Maß begrenzt. Somit gibt das Kontaktelement aufgrund der Blockade gegen eine weitere Einbringung einem Benutzer vorteilhafterweise auch eine über den Reaktantenträger vermittelte haptische Rückmeldung, wenn der Reaktantenträger an den Vorsprung des Kontaktelements anstößt. Die Struktur kann dabei einstückig mit einer Wand der Kammer ausgebildet sein. Insbesondere kann die Struktur zur Fixierung des Reaktantenträgers in der Kammer ausgebildet sein. Beispielsweise kann die Struktur ein oder mehrere Vorsprünge, Ausnehmungen und/oder Wände umfassen, welche den Reaktantenträger zumindest teilweise umschließen und dadurch eine Bewegung insbesondere quer zur Einschieberichtung in die Kammer verhindern. Beispielsweise umfasst die Struktur eine zumindest teilweise U-förmige Umschließung des eingebrachten Reaktantenträgers.

In einer besonders vorteilhaften Weiterbildung der Erfindung umfasst die Vorrichtung einen beweglichen Kolben oder Stempel zur Verdrängung des erzeugten Kalibrierfluids über die erste Schnittstelle. Vorzugsweise ist der Kolben beziehungsweise Stempel entlang einer Längssachse in Richtung der ersten Schnittstelle beweglich, wobei der Kolben beziehungsweise Stempel bevorzugt eine Fläche aufweist, welche eine Querschnittsfläche der bevorzugt quaderförmigen oder zylinderförmigen Kammer quer zu der Längsachse komplett ausfüllt. Dies ermöglicht eine wohldefinierte Verdrängung von zwischen der Fläche des Kolbens beziehungsweise Stempels und der Querschnittsfläche befindlichem Kalibrierfluid durch eine Öffnung der ersten Schnittstelle in den Messpfad der mit der ersten Schnittstelle verbundenen Atemgasanalyseeinrichtung.

Der Vorrichtung kann einen von außerhalb der Vorrichtung bedienbaren Schieber aufweisen, welcher mit dem Kolben beziehungsweise Stempel für eine Bewegung des Kolben beziehungsweise Stempels verbunden ist. Alternativ oder zusätzlich umfasst die Vorrichtung in einer besonderen Ausgestaltung der Erfindung einen Antrieb für die Bewegung des Kolbens beziehungsweise Stempels. Bei dem Antrieb kann es sich insbesondere um einen Elektromotor handeln, welcher mit einer elektrischen Steuerung der Vorrichtung verbunden ist. Dies hat den Vorteil, dass ein Benutzer der Vorrichtung ohne Kraftaufwand eine Verdrängung des Kalibrierfluids aus der Kammer in die Atemgasanalyseeinrichtung bewirken kann. In einer besonders vorteilhaften Weiterbildung der Erfindung umfasst das Kontaktelement einen Heizer zur Erwärmung des ersten Reaktanten. Die Erwärmung kann dabei vorteilhafterweise dazu dienen, eine chemische Reaktion des ersten Reaktanten zur Erzeugung des Kalibrierfluids, insbesondere eines Kalibriergases beispielsweise über eine Oxidation des ersten Reaktanten auszulösen. Der Heizer kann vorteilhafterweise eingerichtet sein, auf eine vorgegebene Reaktionstemperatur aufzuheizen, um eine definierte Temperatur für den ersten Reaktanten bereitzustellen.

Gemäß der Erfindung weist das Kontaktelement eine elektrische leitende Kontaktfläche für einen elektrischen Kontakt mit dem eingebrachten Reaktantenträger auf. Die Vorrichtung kann dazu eingerichtet sein, über den elektrischen Kontakt auf das Vorhandensein eines in die Vorrichtung eingebrachten Reaktantenträger zu erkennen. Ferner kann die Vorrichtung eingerichtet sein, bei einer solchen Erkennung eine Ansteuerung zur Erzeugung des Kalibriergases zu veranlassen, beispielsweise über eine Ansteuerung des Heizers des Kontaktelements. Beispielsweise kann das Kontaktelement eingerichtet sein, über die elektrische Kontaktierung des Reaktantenträgers mit der Kontaktfläche des Kontaktelements einen Stromkreis für die Bestromung des Heizers zu schließen. Dies hat den Vorteil, dass eine Erwärmung des Reaktantenträgers unmittelbar nach dessen Einschub in die erste Öffnung erfolgen kann. Ferner kann die Vorrichtung, insbesondere das Kontaktelement, einen Temperatursensor zur Bestimmung der Temperatur in der Kammer und/oder zur Bestimmung der Temperatur des Reaktantenträgers aufweisen. Vorzugsweise ist das Kontaktelement dabei auch als Temperatursensor ausgestaltet, insbesondere als Widerstandsthermometer. Die Vorrichtung kann dabei eingerichtet sein, über die Bestimmung der Temperatur eine Regelung des Heizers zu steuern.

In einer weiteren vorteilhaften Ausgestaltung der Erfindung umfasst das Kontaktelement einen zweiten Reaktanten. Dies ist insbesondere dann vorteilhaft, wenn die Erzeugung des Kalibrierfluids über eine chemische Reaktion zweier Reaktanten erfolgen soll. Der erste Reaktant wird über den Reaktantenträger eingebracht und mit dem auf dem Kontaktelement vorgelagerten zweiten Reaktanten in Kontakt gebracht. Wenn für die chemische Reaktion erforderlich, können der erste und/oder der zweite Reaktant über den vorzugsweisen Heizer der Vorrichtung auf eine vorgegebene Temperatur zum Start der chemischen Reaktion gebracht werden. Sind für die chemische Reaktion zur Erzeugung des Kalibrierfluids drei Reaktanten erforderlich, können zwei davon, vorzugsweise bereits ineinander vermischt, entweder auf dem Reaktantenträger oder auf dem Kontaktelement und der dritte auf dem Kontaktelement beziehungsweise auf dem Reaktantenträger vorgelagert sein.

In einer weiteren vorteilhaften Ausgestaltung der Erfindung umfasst das Kontaktelement ein Öffnungselement, insbesondere einen Dorn, eine Nadel, ein Vorsprung oder einen Stempel, zum Öffnen eines Behälters des Reaktantenträgers für eine Freisetzung des ersten und/oder zweiten Reaktanten aus dem Behälter. Dies ist insbesondere dann von Vorteil, wenn Reaktanten für die Erzeugung des Kalibrierfluids in flüssiger Form vorliegen. Das Öffnungselement kann vorzugsweise bezüglich der ersten Öffnung derart ausgerichtet sein, dass bei der Einbringung des Reaktantenträgers über die erste Öffnung in die Kammer das Öffnungselement das Öffnen des Behälters bewirkt. Mit anderen Worten bewirkt der Benutzer der Vorrichtung bei einem Einschieben des Reaktantenträgers in die erste Öffnung gleichzeitig auch eine Öffnung des Behälters. Im Fall einer Nadel oder eines Dorns kann sich dazu die Nadel beziehungsweise der Dorn in Richtung der ersten Öffnung erstrecken. Im Falle eines Stempels oder Vorsprungs kann dieser derart angeordnet sein, dass bei einer Krafteinwirkung des Reaktantenträgers gegen den Vorsprung beziehungsweise Stempel der Behälter des Reaktantenträgers aufbricht.

Die Vorrichtung kann in einer vorteilhaften Weiterbildung der Erfindung als Teil einer Ladeeinrichtung oder Ladestation für die Atemgasanalyseeinrichtung ausgebildet sein, insbesondere zur elektrischen Aufladung eines Energiespeichers der Atemgasanalyseeinrichtung. Dies hat den Vorteil, dass die beiden Funktionen des Aufladens und Kalibrierens in einer gemeinsamen Einrichtung kompakt untergebracht werden können. Gegenstand der Erfindung ist somit auch eine Ladeeinrichtung, insbesondere eine Ladestation, für eine Atemgasanalyseeinrichtung umfassend eine erfindungsgemäße Vorrichtung.

Des Weiteren gibt es einen Reaktantenträger für eine chemische Erzeugung eines Kalibrierfluids in einer erfindungsgemäßen Vorrichtung für eine Kalibrierung einer Atemgasanalyseeinrichtung, wobei der Reaktantenträger insbesondere eine Streifenform aufweist, einen ersten Reaktanten und einen Heizer, insbesondere einen Mikrosystem-Heizer, umfasst, wobei der Heizer unterhalb des ersten Reaktanten angeordnet ist. Der Reaktantenträger hat den Vorteil, dass Heizer und daneben angeordneter erster Reaktant gemeinsam für die erfindungsgemäße Vorrichtung bereitgestellt werden. Ferner kann der Reaktantenträger mehrfach verwendet werden, indem neue Reaktanten aufgebracht werden. Außerdem muss in der erfindungsgemäßen Vorrichtung kein Heizer bereitgestellt werden und abhängig von den Reaktanten kann ein Reaktantenträger mit oder ohne Heizer in die Vorrichtung eingebracht werden. Vorzugsweise umfasst der Reaktantenträger eine elektrische Kontaktfläche, welche mit dem Heizer verbunden ist. Der Reaktantenträger kann eingerichtet sein, bei einem elektrischen Kontakt über die Kontaktfläche, also bei Anlegen eines elektrischen Potentials oder einer Bestromung über die Kontaktfläche, den Heizer zu starten. Bevorzugt kann der elektrische Kontakt über die oben beschriebene elektrische Kontaktfläche des Kontaktelements erfolgen.

Wie oben angeführt, ist Gegenstand der Erfindung darüber hinaus ein System nach Anspruch 10 zur Bereitstellung eines Kalibrierfluids, insbesondere eines Stickstoffoxids, für eine Kalibrierung einer Atemgasanalyseeinrichtung. Das System umfasst eine erfindungsgemäße Vorrichtung oder eine erfindungsgemäße Ladeeinrichtung für die Atemgasanalyseeinrichtung umfassend die erfindungsgemäße Vorrichtung. Ferner umfasst das System einen Reaktantenträger, insbesondere den erfindungsgemäßen Reaktantenträger, mit einem ersten Reaktanten zur chemischen Erzeugung des Kalibrierfluids.

Gegenstand der Erfindung ist ferner ein Verfahren nach Anspruch 12 zur Kalibrierung einer

Atemgasanalyseeinrichtung. In einem ersten Schritt wird die Atemgasanalyseeinrichtung mit einer Vorrichtung zur Bereitstellung eines Kalibrierfluids verbunden, wobei die Verbindung der Atemgasanalyseeinrichtung über eine erste Schnittstelle der Vorrichtung mit der Vorrichtung erfolgt. In einem zweiten Schritt wird ein Reaktantenträger mit einem ersten Reaktanten in eine Kammer der Vorrichtung eingebracht. Alternativ kann der zweite Schritt auch vor dem ersten Schritt erfolgen. In einem dritten Schritt wird das Kalibrierfluids, insbesondere umfassend ein oder mehrere verschiedene Stickstoffoxide, über eine chemische Reaktion des ersten Reaktanten in der Kammer erzeugt. In einem vierten Schritt wird das Kalibrierfluid in einen Messpfad der Atemgasanalyseeinrichtung zur Kalibrierung der Atemgasanalyseeinrichtung eingeleitet.

Zu den Vorteilen des erfindungsgemäßen Verfahrens und den folgenden vorteilhaften Weiterbildungen und Ausgestaltungen wird auch auf die oben angeführten korrespondierenden Weiterbildungen und Ausgestaltungen der erfindungsgemäßen Vorrichtung verwiesen.

In einer besonders vorteilhaften Ausgestaltung des Verfahrens wird das Einleiten des Kalibrierfluids in den Messpfad über ein Ansaugen durch eine Pumpe der Atemgasanalyseeinrichtung unterstützt.

Gemäß einer vorteilhaften Weiterbildung des Verfahrens wird zumindest ein Teil des Reaktantenträgers, welcher den ersten Reaktanten umfasst, über einen Heizer der Vorrichtung auf eine Reaktionstemperatur erwärmt.

In einer weiteren besonders vorteilhaften Ausgestaltung des Verfahrens wird über den Reaktantenträger in die Kammer eingebrachtes Kupfernitrat für die Erzeugung von Stickstoffdioxid als Kalibrierfluid durch den Heizer erhitzt. Die Verwendung von Kupfernitrat hat den Vorteil, dass zur Erzeugung von Stickstoffdioxid Kupfernitrat ohne Zugabe weiterer Stoffe nur erhitzt werden muss, vorzugsweise auf 180 °C bei Normaldruck.

Gemäß einer vorteilhaften Weiterbildung des Verfahrens wird das Verfahren mit verschiedene Reaktantenträger mit unterschiedlichen Mengen an erstem Reaktanten wiederholt. Dies hat den Vorteil, dass die Kalibrierung der Atemgasanalyseeinrichtung über einen breiten Messbereich geprüft und gegebenenfalls angepasst werden kann. Kurze Beschreibung der Zeichnungen

Beispiele sind in den Zeichnungen schematisch dargestellt und in der nachfolgenden Beschreibung näher erläutert. Für die in den verschiedenen Figuren dargestellten und ähnlich wirkenden Elemente werden gleiche Bezugszeichen verwendet, wobei auf eine wiederholte Beschreibung der Elemente verzichtet wird.

Es zeigen
Figuren 1 bis 4 schematische Darstellungen von Vorrichtungen zur Bereitstellung eines Kalibrierfluids.
Figur 5 eine schematische Darstellung des Reaktantenträgers und
Figur 6 ein Flussdiagramm des Verfahrens.

### Ausführungsformen der Erfindung

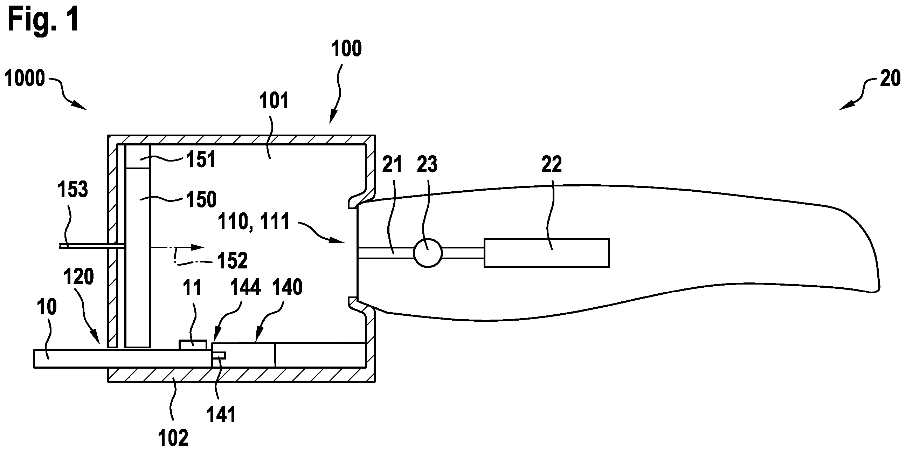

Figur 1 zeigt ein Ausführungsbeispiel einer erfindungsgemäßen Systems 1000 umfassend ein Ausführungsbeispiel einer erfindungsgemäßen Vorrichtung 100 zur Bereitstellung eines Kalibrierfluids, insbesondere eines Stickstoffoxids, für eine Kalibrierung einer Atemgasanalyseeinrichtung 20.

Die Vorrichtung 100 umfasst eine erste Schnittstelle 110 zum Verbinden einer Kammer 101 der Vorrichtung 100 mit einem Messpfad 21 der Atemgasanalyseeinrichtung 20. Ferner umfasst die Vorrichtung 100 eine erste Öffnung 120 zum Einbringen eines Reaktantenträgers 10 mit einem ersten Reaktanten 11 in die Kammer 101 und ein Kontaktelement 140. Das Kontaktelement 140 ist bezüglich der ersten Öffnung 120 derart in der Kammer 101 angeordnet, dass bei eingebrachtem Reaktantenträger 10 ein Kontakt des Kontaktelements 140 mit dem Reaktantenträger 10 für ein Auslösen einer chemischen Reaktion des ersten Reaktanten 11 zur Erzeugung des Kalibrierfluids ermöglicht ist.

Die Kammer 101 kann dabei ein Volumen, also ein Fassungsvermögen für das Kalibrierfluid, von beispielsweise 500 Milliliter aufweisen. Beispielsweise hat die Kammer 101 eine zylindrische Form mit einem Radius von 6 oder 5 Zentimeter und einer Höhe von 4,4 beziehungsweise 6,4 Zentimeter. Alternativ kann die Kammer 101 eine Quaderform aufweisen, mit einer beispielhaften Grundfläche von 7 mal 7 Zentimeter oder 8 mal 8 Zentimeter und einer Höhe von 10,2 beziehungsweise 7,8 Zentimeter. Die Wände der Kammer 101 können einen hitzebeständigen Kunststoff umfassen, beispielsweise Polyetheretherketon (PEEK), Polyphenylensulfid (PPS) und/oder Polytetrafluorethylen (PTFE). Die Kammer 101 kann alternativ oder zusätzlich auch eine das Volumen der Kammer 101 begrenzende Beschichtung für eine Hitzebeständigkeit gegenüber der chemischen Reaktion aufweisen, beispielsweise ebenfalls PEEK, PPS und/oder PTFE. Für eine zusätzliche Kühlfunktion können die Wände der Kammer 101 Kühlrippen oder Fluidkanäle für eine Kühlfluid, beispielsweise Luft, umfassen.

Wie in Figur 1 dargestellt, kann die erste Öffnung 120 insbesondere als Schlitz für das Einbringen eines streifenförmigen Reaktantenträgers 10 ausgebildet sein. Vorzugsweise ist die erste Öffnung 120 verschließbar, wenn kein Reaktantenträger 10 eingeführt ist, beispielsweise über eine Klappe.

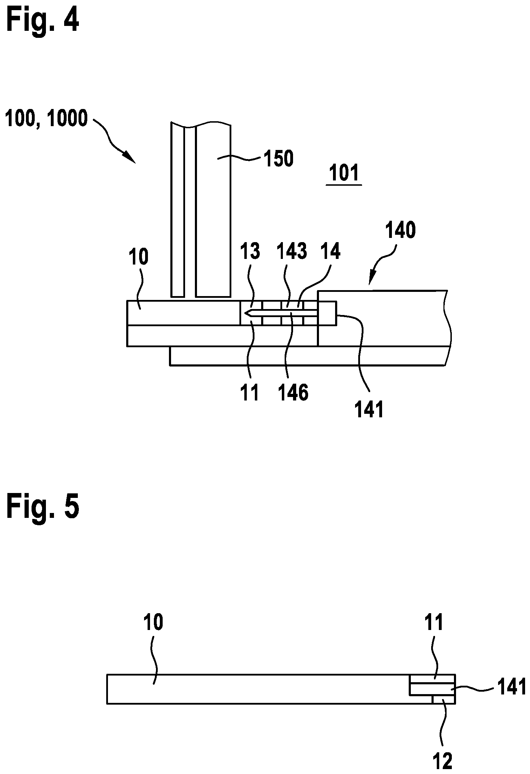

Der Reaktantenträger 10 weist, wie auch in Figur 5 dargestellt, eine Streifenform auf, beispielsweise wie ein aus der Medizintechnik bekannter Teststreifen. Er umfasst in diesem Beispiel ein Trägermaterial, beispielsweise aus Kunststoff, auf dessen einem Ende der erste Reaktant 11 aufgebracht ist, beispielsweise wie dargestellt in einer Vertiefung. Beispielsweise weist der Streifen eine Länge von 50 Millimeter, eine Breite von 5 mm und eine Höhe von 1 Millimeter auf. Vorzugsweise sind die Abmaße der ersten Öffnung 120 auf diese Breite und Höhe angepasst.

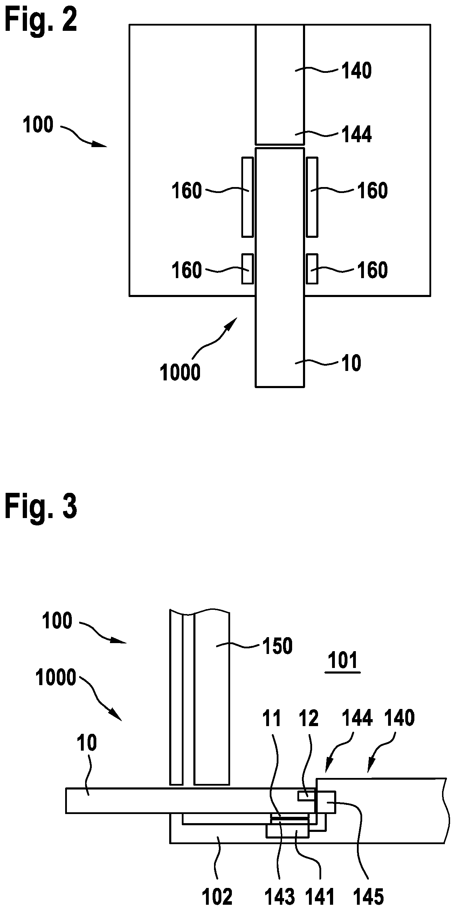

Figur 1 zeigt ferner, dass das Kontaktelement 140 eine Struktur in Form eines Vorsprungs 144 als Begrenzung für eine Einführung des Reaktantenträgers 10 umfasst. Beispielsweise hat der Vorsprung 144 die gleiche Breite wie der Reaktantenträger 10. Figur 2 zeigt eine Draufsicht auf das Kontaktelement 140 und den Reaktantenträgers 10, aus welcher hervorgeht, dass das Kontaktelement 140 weitere Strukturen 160, insbesondere Wände oder Vorsprünge zur Fixierung des Reaktantenträgers 10 in der Kammer 101 umfasst. Wie dargestellt, können diese Strukturen 160 den Reaktantenträger 10 dabei zumindest teilweise U-förmig umschließen, um eine laterale Bewegung des Reaktantenträgers 10 bezüglich der ersten Öffnung 120 zu verhindern. Beispielsweise haben die Strukturen eine Dicke von einigen Millimeter und eine zum Reaktantenträger 10 vergleichbare Höhe von einigen Millimetern. Die Strukturen 144, 160 können die gleichen Kunststoffe wie die Wände der Kammer 101 umfassen und beispielsweise mit einer Wand 102 der Kammer 101 einstückig ausgeführt sein.

Das Kontaktelement 140 umfasst in diesem Ausführungsbeispiel ferner einen Heizer 141 zur Erwärmung eines Teils des Reaktantenträgers 10, insbesondere jenes Teils, welcher den ersten Reaktanten 11 umfasst. Bei dem Heizer 141 kann es sich beispielsweise um einen Widerstandsheizer kandeln. Insbesondere kann es sich bei dem Heizer um einen aus DE 10 2009 056 338 A1 bekannten DSC-Chip handeln. Alternativ kann auch der Reaktantenträger 10 den Heizer 141 umfassen, wie in Figur 5 gezeigt. Der Heizer 141 ist dabei unterhalb des ersten Reaktanten 11 angeordnet.

Bei dem ersten Reaktanten kann es sich beispielsweise um Kupfernitrat ((Cu(NO₃)₂)) in fester Form handeln, welches durch eine Erhitzung auf beispielsweise 180 °C in Kupferoxid, Sauerstoff und Stickstoffdioxid zerfällt. Das dabei erzeugte Stickstoffdioxid bildet das Kalibrierfluid in diesem Beispiel. In Atemluft liegt Stickstoffmonoxid in einer Konzentration von etwa maximal 200 ppb (parts per billion) vor. Für eine Kalibrierung oder eine Überprüfung der Kalibrierung der Atemgasanalyseeinrichtung 20, welche in diesem Beispiel auf der in EP 1384069 B1 offenbarten Vorrichtung basiert, muss eine ähnliche Konzentration bereitgestellt werden, um eine Messung im tatsächlichen Messbereich durchführen zu können. Um zur Bestimmung einer Kalibrierkurve eine Interpolation (und keine Extrapolation) durchführen zu können, ist für die Überprüfung vorzugsweise Stickstoffdioxid in einer Konzentration von 200 ppb, das heißt am oberen Rand des Messbereichs des Gassensors der Atemgasanalyseeinrichtung 20 bereitzustellen. Wie oben beschrieben, beträgt das Volumen der Kammer 101 beispielsweise 500 Milliliter. Es wird vereinfacht angenommen, dass dieses Volumen mit Luft gefüllt ist. Dann lässt sich die nötige Kupfernitrat-Schichtdicke bei einer Grundfläche von 1 Quadratmillimeter folgendermaßen abschätzen. 1 Mol Luft entspricht 6,022*1023 Teilchen und einem Volumen von 22,4 I. 500 Milliliter Luft entsprechen damit 1,34*1022 Teilchen. Wie oben beschrieben sollen 200 ppb davon Stickstoffdioxid sein, was wiederum 2,69*10^15 Teilchen oder 4,46*10^-9 Mol entspricht. Aus 1 Mol Kupfernitrat entstehen bei Erhitzung auf 180 °C 2 Mol Stickstoffdioxid. Um also 4,46*10^-9 Mol Stickstoffdioxid erzeugen zu können, werden 2,23*10^-9 Mol Kupfernitrat benötigt. Die Molmasse von Kupfernitrat beträgt 187,5558 Gramm pro Mol. 2,23*10^-9 Mol Kupfernitrat entsprechen demnach 4,19*10^-7 Gramm. Bei einer Dichte von 3,05 Gramm pro Kubikzentimeter ergibt sich hierfür ein Volumen von 1,37*10^-7 Kubikzentimeter. Bei der oben angenommenen Grundfläche von lQuadratmillimeter ergibt sich eine Schichtdicke von 1,37*10^-5 Zentimeter, das sind 0,137 Mikrometer.

Die Schichtdicke des ersten Reaktanten 11, insbesondere Kupfernitrat wie oben beschrieben, kann variiert werden, um Kalibrierfluid, insbesondere Kalibriergas umfassend Stickstoffdioxid, in unterschiedlichen Konzentrationen bereitzustellen. Beispielsweise können verschiedene Reaktantenträger 10 mit unterschiedlichen Mengen an erstem Reaktanten 11 verwendet werden, um die Kalibrierung des Sensors der Atemgasanalyseeinrichtung 20 über einen breiten Messbereich zu prüfen und gegebenenfalls anzupassen.

Wie in Figur 1 ferner gezeigt, ist die erste Schnittstelle 110 als teilweise formschlüssiger Anschluss für eine möglichst luftdichte Verbindung mit der Atemgasanalyseeinrichtung 20 ausgebildet. Die erste Schnittstelle 110 umfasst dabei eine zweite Öffnung 111, welche vorzugsweise verschließbar ist, beispielsweise über eine Kappe. In der Kammer 101 vorhandenes Fluid, insbesondere das erzeugte Kalibrierfluid, kann somit über die zweite Öffnung in den Messpfad 21 der Atemgasanalyseeinrichtung 20 eintreten. Vorzugsweise umfasst die Atemgasanalyseeinrichtung 20 eine im Messpfad 21 angeordnete Pumpe 23, welche eingerichtet ist, das Fluid über den Messpfad 21 anzusaugen und in die Messkammer 22 weiterzuleiten, in welcher sich der Gassensor 24 der Atemgasanalyseeinrichtung 20 befindet.

Die Vorrichtung 100 umfasst ferner einen beweglichen Kolben 150 oder Stempel 150, im folgenden Stempel 150 genannt, zur Verdrängung des erzeugten Kalibrierfluids über die zweite Öffnung 111 in den Messpfad 21. Der Stempel 150 weist eine Fläche auf, welche eine Querschnittsfläche der in diesem Beispiel quaderförmigen Kammer 101 quer zu einer Längsachse 152 komplett ausfüllt, entlang welcher der Stempel 150 bewegbar ist. Die Bewegung des Stempels kann dabei vorzugsweise über einen elektrischen Antrieb 151 erfolgen, beispielsweise über einen Elektromotor. Alternativ oder zusätzlich kann die Vorrichtung 100 einen von außerhalb der Vorrichtung 100 bedienbaren Schieber 153 aufweisen, welcher mit dem Stempel 150 verbunden ist und von einem Benutzer zur Bewegung des Stempels 150 benutzt werden kann. Eine Wand 102 der Kammer 101 kann dabei eine Ausnehmung für die Aufnahme des Reaktantenträgers 10 und das daran bündig anschließende Kontaktelement 140 aufweisen, so dass der Stempel 150 über den Reaktantenträger 10 und das Kontaktelement 140 für die Verdrängung des erzeugten Kalibrierfluids über die erste Schnittstelle 110 hinwegbewegt werden kann. Alternativ kann der Stempel 150 eine auf die Abmaße des Reaktantenträger 10 und des Kontaktelements 140 angepasste Ausnehmung aufweisen.

Figur 3 zeigt ein weiteres Ausführungsbeispiel des erfindungsgemäßen Systems 1000 und der erfindungsgemäßen Vorrichtung 100. Es unterscheidet sich von dem Ausführungsbeispiel gemäß Figur 1 dadurch, dass der Heizer 141 des Kontaktelements 140 derart in einer Wand 102, in diesem Beispiel im Boden 102, der Kammer 101 derart angeordnet ist, dass bei einem Einschub des Reaktantenträgers 10 bis zum von der Wand abstehenden Vorsprung 144 des Kontaktelements 140 der Heizer sich lateral vom dem den ersten Reaktanten 11 aufweisenden Ende des Reaktantenträgers 10 befindet. Auf dem Heizer 143 ist in diesem Beispiel ein zweiter Reaktant 143 angeordnet, so dass der erste Reaktant 11 und der zweite Reaktant 143 in Kontakt kommen können. In diesem Ausführungsbeispiel kann es sich um zwei Reaktanten handeln, welche bei einer chemischen Reaktion miteinander das Kalibrierfluid freisetzen, insbesondere ein Stickstoffoxid wie beispielsweise Stickstoffdioxid. Vorteilhafterweise kann der Heizer 141 dabei die Reaktion durch Erwärmung insbesondere des zweiten Reaktanten 143 unterstützen. Bei dem ersten Reaktanten kann es sich beispielsweise um Eisen(II)-Sulfat und bei dem zweiten Reaktanten um eine Mischung aus Natriumbromid und Natriumnitrit handeln, welche in einer chemischen Reaktion miteinander ein Gasgemisch umfassend zu etwa 98,8 % Stickstoffmonoxid und zu etwa 1,2 % Stickstoff erzeugen.

In einer alternativen Ausgestaltung ist kein zweiter Reaktant 143 auf dem Heizer 141 angeordnet und der Heizer 141 kann eine Erwärmung des ersten Reaktanten 11 zur Erzeugung des Kalibrierfluids bewirken, beispielsweise eine Erwärmung von Kupfernitrat zur Erzeugung von Stickstoffdioxid.

In einer weiteren alternativen Ausgestaltung ist kein Heizer 141 unterhalb des zweiten Reaktanten 143 angeordnet. Dies ist insbesondere dann von Vorteil, wenn für die chemische Reaktion der beiden Reaktanten 11, 143 keine externe Wärme benötigt wird.

In der in Figur 3 gezeigten Vorrichtung 100 weist das Kontaktelement 140 ferner eine elektrische leitende Kontaktfläche 145 für einen elektrischen Kontakt mit dem eingebrachten Reaktantenträger 11 auf. Die Vorrichtung 100 kann dazu eingerichtet sein, über den elektrischen Kontakt auf das Vorhandensein eines in die Vorrichtung eingebrachten Reaktantenträger 11 zu erkennen. Dazu weist der Reaktantenträger 11 vorzugsweise ebenfalls eine elektrische leitende Kontaktfläche 12 oder ein elektrisches Kontaktelement 12 auf, wie in Figur 5 gezeigt. Ferner kann die Vorrichtung 100 eingerichtet sein, bei einer solchen Erkennung eine Ansteuerung zur Erzeugung des Kalibriergases zu veranlassen, beispielsweise über eine Ansteuerung der Heizers 141 des Kontaktelements 140. Beispielsweise kann auch das Kontaktelement 140 eingerichtet sein, über die elektrische Kontaktierung des Reaktantenträgers 11 mit der Kontaktfläche des Kontaktelements einen Stromkreis für die Bestromung des Heizers 141 zu schließen.

Wenn der Reaktantenträger 10 den Heizer 141 umfasst, kann die elektrische Kontaktfläche 12 beziehungsweise das elektrische Kontaktelement 12 wie in Figur 5 gezeigt mit dem Heizer 141 verbunden sein. Der Reaktantenträger 10 kann dabei eingerichtet sein, bei einem elektrischen Kontakt über die Kontaktfläche 12, also bei Anlegen eines elektrischen Potentials oder einer Bestromung über die Kontaktfläche 12, den Heizer 141 zu starten. Bevorzugt kann der elektrische Kontakt über die oben beschriebene elektrische Kontaktfläche 145 des Kontaktelements 140 erfolgen.

Figur 4 zeigt ein System 1000 und reine Vorrichtung 100. Es unterscheidet sich von den Ausführungsbeispielen gemäß Figur 1 und Figur 3 dadurch, dass das Kontaktelement 140 ein Öffnungselement 146 in der Form eines Dorns 146 oder einer Nadel 146 aufweist. Das Öffnungselement 146 kann wie dargestellt vorzugsweise bezüglich der ersten Öffnung 120 derart ausgerichtet sein, dass bei der Einbringung des Reaktantenträgers 10 über die erste Öffnung 120 in die Kammer das Öffnungselement 146 das Öffnen eines oder mehrerer Behälter 13, 14 des Reaktantenträgers 10 bewirkt. Wie in Figur 4 ersichtlich, erstreckt sich der Dorn 146 beziehungsweise die Nadel 146 einem spitzen Ende in Richtung der ersten Öffnung 120. Somit bewirkt ein Benutzer der Vorrichtung 100 bei einem Einschieben des Reaktantenträgers 10 in die erste Öffnung 120 gleichzeitig auch eine Öffnung der Behälter 13, 14 des Reaktantenträgers. In diesem Beispiel umfasst der Reaktantenträger einen ersten Behälter 13 mit dem ersten Reaktanten 11 und einen zweiten Behälter 14 mit dem zweiten Reaktanten 143, welche beim Einschieben nacheinander von dem Öffnungselement 146 durchstochen werden. So können die beiden Reaktanten 11, 143 aus den Behältern 13, 14 austreten und sich für die nachfolgende chemische Reaktion zur Erzeugung des Kalibrierfluids vermischen. Diese Ausführungsform der Erfindung ist somit insbesondere für flüssige Reaktanten 11, 143 von Vorteil.

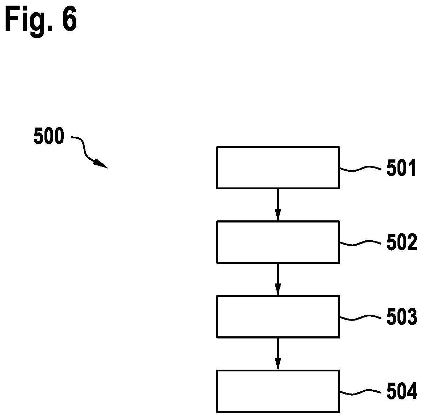

Figur 6 zeigt ein Flussdiagramm des Verfahrens 500 zur Kalibrierung einer Atemgasanalyseeinrichtung. Das Verfahren 500 kann dabei mit einem der oben genannten Ausführungsbeispiele des erfindungsgemäßen Systems 1000 und der erfindungsgemäßen Vorrichtung 100 durchgeführt werden. In einem ersten Schritt 501 wird die Atemgasanalyseeinrichtung 20 mit der Vorrichtung 100 zur Bereitstellung eines Kalibrierfluids verbunden, wobei die Verbindung der Atemgasanalyseeinrichtung 20 über die erste Schnittstelle der Vorrichtung 100 mit der Vorrichtung 100 erfolgt. In einem zweiten Schritt 502 wird ein Reaktantenträger 10 mit dem ersten Reaktanten 11 in die Kammer 101 der Vorrichtung 100 eingebracht. Alternativ kann der zweite Schritt 502 auch vor dem ersten Schritt 501 erfolgen. In einem dritten Schritt 503 wird das Kalibrierfluids, insbesondere umfassend ein oder mehrere verschiedene Stickstoffoxide, über eine chemische Reaktion umfassend zumindest den ersten Reaktanten 11 in der Kammer 101 erzeugt. In einem vierten Schritt 504 wird das Kalibrierfluid in den Messpfad 21 der Atemgasanalyseeinrichtung 20 zur Kalibrierung der Atemgasanalyseeinrichtung 20 eingeleitet und die Kalibrierung durchgeführt. Vorzugsweise wird das Einleiten des Kalibrierfluids in den Messpfad 21 über ein Ansaugen durch die Pumpe 23 der Atemgasanalyseeinrichtung 20 unterstützt. Gemäß einer vorteilhaften Weiterbildung des Verfahrens wird zumindest ein Teil des Reaktantenträgers 10, welcher den ersten Reaktanten 11 umfasst, über den Heizer 141 der Vorrichtung 100 auf eine Reaktionstemperatur erwärmt. Insbesondere kann es sich bei dem ersten Reaktanten 11 wie oben beschrieben um Kupfernitrat handeln und somit die Erwärmung vorzugsweise auf mindestens 180 °C erfolgen.

Die Erfindung ist in den Ansprüchen definiert.

## Patentansprüche

1. Vorrichtung (100) zur Bereitstellung eines Kalibrierfluids, insbesondere eines Stickstoffoxids, für eine Kalibrierung einer Atemgasanalyseeinrichtung (20), umfassend eine erste Schnittstelle (110) zum Verbinden einer Kammer (101) der Vorrichtung (100) mit einem Messpfad (21) der Atemgasanalyseeinrichtung (20), eine erste Öffnung (120) zum Einbringen eines Reaktantenträgers (10) mit einem ersten Reaktanten (11) in die Kammer (101) und ein Kontaktelement (140) mit einer elektrisch leitenden Kontaktfläche (145) für einen elektrischen Kontakt mit dem eingebrachten Reaktantenträger (10), wobei das Kontaktelement (140) bezüglich der ersten Öffnung (120) derart in der Kammer (101) angeordnet ist, dass bei eingebrachtem Reaktantenträger (10) ein elektrischer Kontakt des Kontaktelements (140) mit dem Reaktantenträger (10) für ein Auslösen einer chemischen Reaktion des ersten Reaktanten (11) zur Erzeugung des Kalibrierfluids eingerichtet ist.

2. Vorrichtung (100) nach Anspruch 1, wobei das Kontaktelement (140) eine Struktur (144), insbesondere einen Vorsprung (144), zur Kontaktierung des eingebrachten Reaktantenträgers (10) umfasst.

3. Vorrichtung (100) nach Anspruch 2, wobei die Struktur (144, 160) zur Fixierung des Reaktantenträgers (10) in der Kammer ausgebildet ist.

4. Vorrichtung (100) nach einem der vorhergehenden Ansprüche, wobei die Vorrichtung (100) einen beweglichen Kolben (150) oder Stempel (150) zur Verdrängung des erzeugten Kalibrierfluids über die erste Schnittstelle (110) umfasst.

5. Vorrichtung (100) nach einem der vorhergehenden Ansprüche, wobei das Kontaktelement (140) einen Heizer (141) zur Erwärmung des ersten Reaktanten (11) auf eine vorgegebene Reaktionstemperatur umfasst.

6. Vorrichtung (100) nach einem der vorhergehenden Ansprüche, wobei das Kontaktelement (140) die elektrische leitende Kontaktfläche (145) für einen elektrischen Kontakt des eingebrachten Reaktantenträger (10) mit dem Heizer (141) aufweist.

7. Vorrichtung (100) nach einem der vorhergehenden Ansprüche, wobei das Kontaktelement (140) einen zweiten Reaktanten (143) umfasst.

8. Vorrichtung (100) nach einem der vorhergehenden Ansprüche, wobei das Kontaktelement (140) ein Öffnungselement (146), insbesondere einen Dorn oder einen Vorsprung, zum Öffnen eines Behälters (13, 14) des Reaktantenträgers (10) für eine Freisetzung des ersten und/oder zweiten Reaktanten (11, 12) aus dem Behälter umfasst.

9. Ladeeinrichtung (100), insbesondere Ladestation, zur elektrischen Aufladung eines Energiespeichers einer Atemgasanalyseeinrichtung (20), umfassend eine Vorrichtung (100) nach einem der vorhergehenden Ansprüche.

10. System (1000) zur Bereitstellung eines Kalibrierfluids, insbesondere eines Stickstoffoxids, für eine Kalibrierung einer Atemgasanalyseeinrichtung (20), umfassend eine Vorrichtung (100) nach einem der Ansprüche 1 bis 8 oder eine Ladeeinrichtung (100) nach Anspruch 9 und einen Reaktantenträger (10) mit einem ersten Reaktanten (11) zur chemischen Erzeugung des Kalibrierfluids.

11. System (1000) nach Anspruch 10, wobei der Reaktantenträger (10) einen ersten Reaktanten (11) und einen Heizer (141), insbesondere einen Mikrosystem-Heizer, umfasst, wobei der Heizer (141) unterhalb des ersten Reaktanten (11) angeordnet ist.

12. Verfahren (500) zur Kalibrierung einer Atemgasanalyseeinrichtung (20), umfassend die Schritte:
• Verbinden (501) der Atemgasanalyseeinrichtung (20) mit einer Vorrichtung (100) zur Bereitstellung eines Kalibrierfluids, wobei die Atemgasanalyseeinrichtung (20) über eine erste Schnittstelle (110) der Vorrichtung (100) mit der Vorrichtung (100) verbunden wird.
• Einbringen (502) eines Reaktantenträgers (10) mit einem ersten Reaktanten (11) in eine Kammer (101) der Vorrichtung (100), wobei beim Einbringen (502) ein elektrischer Kontakt einer elektrischen Kontaktfläche (145) eines Kontaktelements (140) der Vorrichtung (100) mit dem Reaktantenträger (10) für ein Auslösen einer chemischen Reaktion des ersten Reaktanten (11) zur Erzeugung des Kalibrierfluids hergestellt wird.
• Erzeugen (503) des Kalibrierfluids, insbesondere eines Stickstoffoxids, über die chemische Reaktion des ersten Reaktanten (11) in der Kammer (101).
• Einleiten des (504) Kalibrierfluids in einen Messpfad (21) der Atemgasanalyseeinrichtung (20) und Durchführung (504) der Kalibrierung der Atemgasanalyseeinrichtung (20)

13. Verfahren (500) nach Anspruch 12, wobei Einleiten in den Messpfad (21) über ein Ansaugen durch eine Pumpe (23) der Atemgasanalyseeinrichtung (20) unterstützt wird.

14. Verfahren (500) nach Anspruch 12 oder 13, wobei zumindest ein Teil des Reaktantenträgers (10), welcher den ersten Reaktanten (11) umfasst, über einen Heizer (141) der Vorrichtung (100) auf eine Reaktionstemperatur erwärmt wird.

15. Verfahren (500) nach einem der Ansprüche 12 bis 14, wobei über den Reaktantenträger (10) in die Kammer (101) eingebrachtes Kupfernitrat als erster Reaktant (11) für die Erzeugung von Stickstoffdioxid als Kalibriergas durch den Heizer (141) erhitzt wird.

## Claims

1. Apparatus (100) for providing a calibration fluid, in particular a nitrogen oxide, for calibration of a respiratory-gas-analysis device (20), comprising a first interface (110) for connecting a chamber (101) of the apparatus (100) to a measurement path (21) of the respiratory-gas-analysis device (20), a first opening (120) for introduction of a reactant carrier (10) with a first reactant (11) into the chamber (101), and a contact element (140) with an electrically conductive contact surface (145) for an electrical contact with the introduced reactant carrier (10), wherein the contact element (140) is arranged in the chamber (101) in relation to the first opening (120) in such a way that, when the reactant carrier (10) has been introduced, an electrical contact of the contact element (140) with the reactant carrier (10) is configured to trigger a chemical reaction of the first reactant (11) to produce the calibration fluid.

2. Apparatus (100) according to Claim 1, wherein the contact element (140) comprises a structure (144), in particular a projection (144), for contacting the introduced reactant carrier (10).

3. Apparatus (100) according to Claim 2, wherein the structure (144, 160) is configured for fixing the reactant carrier (10) in the chamber.

4. Apparatus (100) according to one of the preceding claims, wherein the apparatus (100) comprises a movable piston (150) or plunger (150) for displacing the produced calibration fluid via the first interface (110).

5. Apparatus (100) according to one of the preceding claims, wherein the contact element (140) comprises a heater (141) for heating the first reactant (11) to a predefined reaction temperature.

6. Apparatus (100) according to one of the preceding claims, wherein the contact element (140) has the electrically conductive contact surface (145) for an electrical contact of the introduced reactant carrier (10) with the heater (141).

7. Apparatus (100) according to one of the preceding claims, wherein the contact element (140) comprises a second reactant (143).

8. Apparatus (100) according to one of the preceding claims, wherein the contact element (140) comprises an opening element (146), in particular a pin or a projection, for opening a container (13, 14) of the reactant carrier (10) for release of the first and/or second reactant (11, 12) from the container.

9. Charging device (100), in particular charging station, for electrical charging of an energy store of a respiratory-gas-analysis device (20), comprising an apparatus (100) according to one of the preceding claims.

10. System (1000) for providing a calibration fluid, in particular a nitrogen oxide, for calibration of a respiratory-gas-analysis device (20), comprising an apparatus (100) according to one of Claims 1 to 8 or a charging device (100) according to Claim 9, and a reactant carrier (10) with a first reactant (11) for chemical production of the calibration fluid.

11. System (1000) according to Claim 10, wherein the reactant carrier (10) comprises a first reactant (11) and a heater (141), in particular a microsystem heater, wherein the heater (141) is arranged below the first reactant (11).

12. Method (500) for calibrating a respiratory-gas-analysis device (20), comprising the steps of:
• connecting (501) the respiratory-gas-analysis device (20) to an apparatus (100) for providing a calibration fluid, wherein the respiratory-gas-analysis device (20) is connected to the apparatus (100) via a first interface (110) of the apparatus (100),
• introducing (502) a reactant carrier (10) with a first reactant (11) into a chamber (101) of the apparatus (100), wherein the introducing (502) results in an electrical contact of an electrical contact surface (145) of a contact element (140) of the apparatus (100) with the reactant carrier (10) for triggering a chemical reaction of the first reactant (11) to produce the calibration fluid being established,
• producing (503) the calibration fluid, in particular a nitrogen oxide, via the chemical reaction of the first reactant (11) in the chamber (101),
• introducing (504) the calibration fluid into a measurement path (21) of the respiratory-gas-analysis device (20) and carrying out (504) the calibration of the respiratory-gas-analysis device (20).

13. Method (500) according to Claim 12, wherein introducing into the measurement path (21) is assisted via a sucking-in action of a pump (23) of the respiratory-gas-analysis device (20).

14. Method (500) according to Claim 12 or 13, wherein at least a part of the reactant carrier (10), comprising the first reactant (11), is heated via a heater (141) of the apparatus (100) to a reaction temperature.

15. Method (500) according to one of Claims 12 to 14, wherein copper nitrate introduced into the chamber (101) via the reactant carrier (10) is heated by the heater (141) as first reactant (11) for producing nitrogen dioxide as calibration gas.

## Revendications

1. Dispositif (100) pour la fourniture d'un fluide d'étalonnage, notamment d'un oxyde d'azote, pour un étalonnage d'un appareil d'analyse de gaz respiratoire (20), comprenant une première interface (110) pour la liaison d'une chambre (101) du dispositif (100) à une voie de mesure (21) de l'appareil d'analyse de gaz respiratoire (20), une première ouverture (120) pour l'introduction d'un support de réactif (10) avec un premier réactif (11) dans la chambre (101) et un élément de contact (140) avec une surface de contact électriquement conductrice (145) pour un contact électrique avec le support de réactif introduit (10), l'élément de contact (140) étant agencé dans la chambre (101) par rapport à la première ouverture (120) de telle sorte que, lorsque le support de réactif (10) est introduit, un contact électrique de l'élément de contact (140) avec le support de réactif (10) est établi pour déclencher une réaction chimique du premier réactif (11) pour la production du fluide d'étalonnage.

2. Dispositif (100) selon la revendication 1, dans lequel l'élément de contact (140) comprend une structure (144), notamment une protubérance (144), pour la mise en contact avec le support de réactif introduit (10).

3. Dispositif (100) selon la revendication 2, dans lequel la structure (144, 160) est configurée pour la fixation du support de réactif (10) dans la chambre.

4. Dispositif (100) selon l'une quelconque des revendications précédentes, dans lequel le dispositif (100) comprend un piston (150) ou poinçon (150) mobile pour le refoulement du fluide d'étalonnage produit par l'intermédiaire de la première interface (110).

5. Dispositif (100) selon l'une quelconque des revendications précédentes, dans lequel l'élément de contact (140) comprend un dispositif de chauffage (141) pour le chauffage du premier réactif (11) à une température de réaction prédéterminée.

6. Dispositif (100) selon l'une quelconque des revendications précédentes, dans lequel l'élément de contact (140) présente la surface de contact électriquement conductrice (145) pour un contact électrique du support de réactif introduit (10) avec le dispositif de chauffage (141).

7. Dispositif (100) selon l'une quelconque des revendications précédentes, dans lequel l'élément de contact (140) comprend un deuxième réactif (143).

8. Dispositif (100) selon l'une quelconque des revendications précédentes, dans lequel l'élément de contact (140) comprend un élément d'ouverture (146), notamment un mandrin ou une protubérance, pour l'ouverture d'un récipient (13, 14) du support de réactif (10) pour une libération du premier et/ou du deuxième réactif (11, 12) hors du récipient.

9. Appareil de charge (100), notamment station de charge, pour la charge électrique d'un accumulateur d'énergie d'un appareil d'analyse de gaz respiratoire (20), comprenant un dispositif (100) selon l'une quelconque des revendications précédentes.

10. Système (1000) pour la fourniture d'un fluide d'étalonnage, notamment d'un oxyde d'azote, pour un étalonnage d'un appareil d'analyse de gaz respiratoire (20), comprenant un dispositif (100) selon l'une quelconque des revendications 1 à 8 ou un appareil de charge (100) selon la revendication 9 et un support de réactif (10) avec un premier réactif (11) pour la production chimique du fluide d'étalonnage.

11. Système (1000) selon la revendication 10, dans lequel le support de réactif (10) comprend un premier réactif (11) et un dispositif de chauffage (141), notamment un dispositif de chauffage à microsystème, le dispositif de chauffage (141) étant agencé sous le premier réactif (11).

12. Procédé (500) pour l'étalonnage d'un appareil d'analyse de gaz respiratoire (20), comprenant les étapes suivantes :
- la liaison (501) de l'appareil d'analyse de gaz respiratoire (20) à un dispositif (100) pour la fourniture d'un fluide d'étalonnage, l'appareil d'analyse de gaz respiratoire (20) étant relié au dispositif (100) par l'intermédiaire d'une première interface (110) du dispositif (100),
- l'introduction (502) d'un support de réactif (10) avec un premier réactif (11) dans une chambre (101) du dispositif (100) ; lors de l'introduction (502), un contact électrique d'une surface de contact électrique (145) d'un élément de contact (140) du dispositif (100) étant établi avec le support de réactif (10) pour un déclenchement d'une réaction chimique du premier réactif (11) pour la production du fluide d'étalonnage,
- la production (503) du fluide d'étalonnage, notamment d'un oxyde d'azote, par l'intermédiaire de la réaction chimique du premier réactif (11) dans la chambre (101),
- l'introduction du fluide d'étalonnage (504) dans une voie de mesure (21) de l'appareil d'analyse de gaz respiratoire (20) et la réalisation (504) de l'étalonnage de l'appareil d'analyse de gaz respiratoire (20).

13. Procédé (500) selon la revendication 12, dans lequel l'introduction dans la voie de mesure (21) est assistée par une aspiration par une pompe (23) de l'appareil d'analyse de gaz respiratoire (20).

14. Procédé (500) selon la revendication 12 ou 13, dans lequel au moins une partie du support de réactif (10), qui comprend le premier réactif (11), est chauffée à une température de réaction par l'intermédiaire d'un dispositif de chauffage (141) du dispositif (100).

15. Procédé (500) selon l'une quelconque des revendications 12 à 14, dans lequel du nitrate de cuivre introduit dans la chambre (101) par l'intermédiaire du support de réactif (10) est chauffé en tant que premier réactif (11) par le dispositif de chauffage (141) pour la production de dioxyde d'azote en tant que gaz d'étalonnage.
